(19) Europäisches Patentamt
European Patent Office
Office européen des brevets

(11) **EP 3 128 959 B1**

(12) **EUROPEAN PATENT SPECIFICATION**

(45) Date of publication and mention
of the grant of the patent:
**30.09.2020 Bulletin 2020/40**

(51) Int Cl.:
*A61F 2/90* *(2013.01)*     *A61F 2/962* *(2013.01)*

(21) Application number: **15717382.4**

(86) International application number:
**PCT/US2015/024677**

(22) Date of filing: **07.04.2015**

(87) International publication number:
**WO 2015/157258 (15.10.2015 Gazette 2015/41)**

(54) **MEDICAL DEVICES**

MEDIZINISCHE VORRICHTUNGEN

DISPOSITIFS MÉDICAUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.04.2014 US 201461976754 P**

(43) Date of publication of application:
**15.02.2017 Bulletin 2017/07**

(73) Proprietor: **Boston Scientific Scimed, Inc.
Maple Grove, MN 55311 (US)**

(72) Inventor: **HARRIS, Colby
Weston, Massachusetts 02493 (US)**

(74) Representative: **Vossius & Partner
Patentanwälte Rechtsanwälte mbB
Siebertstrasse 3
81675 München (DE)**

(56) References cited:
**EP-A1- 2 510 907        EP-A2- 1 982 677
WO-A1-2013/052528      WO-A2-2006/053270
WO-A2-2013/011502      DE-A1-102011 011 180
US-A1- 2004 204 749    US-A1- 2006 100 688
US-A1- 2013 245 745**

## Description

### TECHNICAL FIELD

The present disclosure pertains to medical devices, systems, and methods for using the medical devices. More particularly, the present disclosure pertains to medical devices such as a stent and a delivery catheter, and methods for using these devices.

### BACKGROUND

Existing stents can be made by braiding one or more wires, for example. These wires can be made from a metallic material having a radial strength such that the stent can be temporarily compressed, for example, when delivered into a patient's body through a delivery system.

In order to more easily deliver the stent to the treatment site, it may be desirable to use a delivery system having the smallest possible diameter or cross-section. It may also be desirable to use a stent having sufficient radial strength to support the body lumen at the treatment site. A stiffer stent, however, can be more difficult to compress into a small diameter for delivery and can lead to an undesirable bulge in the delivery system along a portion of the stent's length.

WO 2006/053270 A2 relates to an implantable stent including a plurality of elongate wires braided to form a hollow tubular structure having a tubular wall to define an interior surface and an exterior surface and having opposed open first and second ends, wherein the opposed open first and second ends are atraumatic ends. The atraumatic ends of the stent are desirably free of any loose wire ends. DE 10 2011 011 180 A1 relates to a medical device which comprises an expandable lattice mesh consisting of intersecting wires which form loops on at least one longitudinal end of the lattice mesh. The disclosed medical device is characterised in that at least one loop is a reinforcement loop formed by a wire which, at least in the region of said reinforcement loop, has a larger cross-sectional diameter than the wires which form the other loops.

Consequently, there remains a need for a stent having a small compressed configuration and also the requisite degree of radial strength. Further, there remains a need for a delivery system that can be used in conjunction with such a stent.

### SUMMARY

In at least one embodiment, a stent comprises a braided wire body. The braided wire has a proximal end and a distal end. Desirably, the braided wire body is formed from a first wire and a second wire having a first cross-sectional area and a second cross-sectional area, respectively, where the second cross-sectional area is different from the first cross-sectional area. The proximal end has a plurality of peaks comprising first peaks and first loop ends. The first loop ends circumferentially alternating with the first peaks and the first loop ends extending proximally beyond the first peaks.

In at least one embodiment, a delivery catheter and stent combination includes a stent having a proximal end and a distal end. The proximal end has a plurality of peaks, the peaks comprising first peaks and first loop ends. The first loop ends circumferentially alternate with the first peaks. The first loop ends extend proximally beyond the first peaks. The delivery catheter comprises a retractable sheath, an inner catheter member, and an anchor member. The anchor member is disposed exterior to the inner catheter member and comprises a plurality of protrusions. The protrusions extend into the first loop ends of the stent. Desirably, the retractable sheath surrounds the stent and anchor member.

The above summary of some embodiments is not intended to describe each disclosed embodiment or every implementation of the present disclosure. The Figures and Detailed Description, which follow, more particularly exemplify these embodiments.

### BRIEF DESCRIPTION OF THE DRAWINGS

A detailed description of the invention is hereafter described with specific reference being made to the drawings.

FIG. 1 is a side-view of an embodiment of a stent.
FIG. 2 is a detailed side-view of a portion of the stent of FIG. 1.
FIGS. 3A and 3B are detailed views of first loop ends of embodiments of the stent.
FIG. 4 is a perspective view of an embodiment of a delivery catheter and stent.
FIG. 5 is a perspective side-view of the delivery catheter and stent of FIG. 4.
FIG. 6 shows a cross-sectional view of an embodiment of a first wire.
FIG. 7 shows a cross-sectional view of an embodiment of a second wire.
FIG. 8 shows a side view of a PRIOR ART stent, taken from US Pub. No. 2006/0116752.

While the disclosure is amenable to various modifications and alternative forms, specifics thereof have been shown by way of example in the drawings and will be described in detail.

## DETAILED DESCRIPTION

The recitation of numerical ranges by endpoints includes all numbers within that range (e.g., 1 to 5 includes 1, 1.5, 2, 2.75, 3, 3.80, 4, and 5).

As used herein, the singular forms "a", "an", and "the" include plural references unless the context clearly indicates otherwise. As used herein, the term "or" is generally employed in its sense including "and/or" unless the context clearly evidences or indicates otherwise.

References herein to "an embodiment," "some embodiments," "other embodiments," etc., indicate that an embodiment includes a particular feature, structure, or characteristic, but not every embodiment necessarily includes the particular feature, structure, or characteristic. Moreover, such phrases do not necessarily refer to the same embodiment. Further, when a particular feature, structure, or characteristic is described in connection with an embodiment (or more embodiments), it should be understood that such feature, structure, or characteristic may also be used in connection with other embodiments, whether or not explicitly described, unless clearly evidenced or stated to the contrary.

The following detailed description should be read with reference to the drawings in which similar elements in different drawings are numbered the same. The drawings, which are not necessarily to scale, depict illustrative embodiments and are not intended to limit the scope of the disclosure.

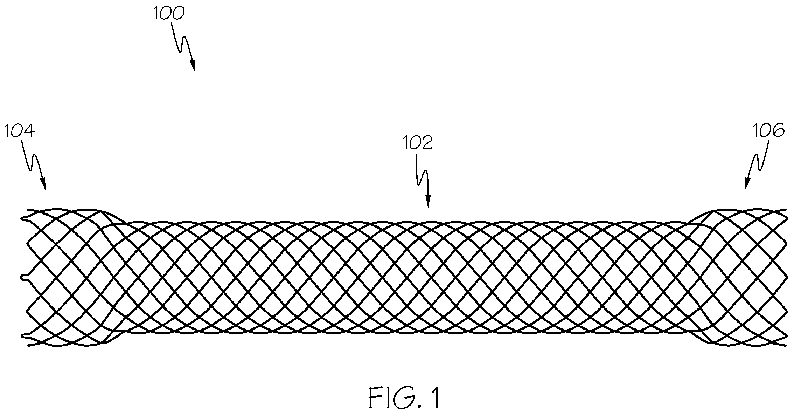

FIG. 1 is a side-view of an embodiment of a stent 100. As shown, in some embodiments, the stent 100 includes a braided wire body 102 having a proximal end 104 and a distal end 106. In some embodiments, the braided wire body 102 has a hollow, tubular structure configured to be deployed inside a body lumen. Generally, the stent 100 is hollow so that body fluid can pass therethrough, once the stent 100 is deployed within the body lumen. Upon deployment, the stent 100 abuts an inner wall of the body lumen, and thus the diameter of the stent 100 is determined based on the diameter of the body lumen in which the stent is to be deployed.

In some embodiments, the wires of braided stent 100 are made up of one or more biocompatible materials. Examples include biocompatible metals or metal alloys, composites, or polymers. In some embodiments, the inner or outer surface of the hollow stent is coated with one or more materials. For example, the stent can have a coating of carbon, platinum, or heparin, which can decrease, impede, or prevent the occurrence of thrombosis.

The biocompatible polymers include synthetic polymeric materials and bioabsorbable or biodegradable polymeric materials. In some embodiments, the wires of the stent 100 are manufactured using biocompatible metals or alloys such as, but not limited to, nitinol, stainless steel, cobalt-based alloy such as Elgiloy, platinum, gold, titanium, tantalum, niobium, polymeric materials and combinations thereof. Exemplary synthetic polymeric materials include, but are not limited to, polyesters, including polyethylene terephthalate (PET) polyesters, polypropylenes, polyethylenes, polyurethanes, polyolefins, polyvinyls, polymethylacetates, polyamides, naphthalane dicarboxylene derivatives, silks, and polytetrafluoroethylenes. The polymeric materials further include a metallic, a glass, ceramic or carbon constituent or fiber. Useful and nonlimiting examples of bioabsorbable or biodegradable polymeric materials include poly(L-lactide) (PLLA), poly(D,L-lactide) (PLA), poly(glycolide) (PGA), poly(L-lactide-co-D,L-lactide) (PLLA/PLA), poly(L-lactide-co-glycolide) (PLLA/PGA), poly(D,L-lactide-co-glycolide) (PLA/PGA), poly(glycolide-co-trimethylene carbonate) (PGA/PTMC), polydioxanone (PDS), Polycaprolactone (PCL), polyhydroxybutyrate (PHBT), poly(phosphazene) poly(D,L-lactide-co-caprolactone) PLA/PCL), poly(glycolide-co-caprolactone) (PGA/PCL), poly(phosphate ester), and the like.

In some embodiments, wires of the stent 100 are made from polymeric materials that include radiopaque materials, such as metallic-based powders or ceramic-based powders, particulates or pastes that may be incorporated into the polymeric material. For example, the radiopaque material may be blended with the polymer composition from which the polymeric wire is formed, and subsequently fashioned into the stent as described herein. Alternatively, the radiopaque material may be applied to the surface of the metal or polymer stent. In either embodiment, various radiopaque materials and their salts and derivatives may be used including, without limitation, bismuth, barium and its salts such as barium sulfate, tantalum, tungsten, gold, platinum and titanium. Metallic complexes useful as radiopaque materials are also contemplated. The stent may be selectively made radiopaque at desired areas along the wire or made be fully radiopaque, depending on the desired end-product and application.

In some embodiments, wires of the stent 100 have an inner core of tantalum, gold, platinum, iridium or a combination of thereof, and an outer member or layer of nitinol to provide a composite wire for improved radiocapicity or visibility. The inner core may be platinum, and the outer layer may be nitinol, for example. In some embodiments, the inner core of platinum represents about at least 10% of the wire based on the overall cross-sectional area. Moreover, nitinol having shape memory properties and ninitol sans shape memory properties can be employed in formation of the wire. The wires may be made from nitinol, or a composite wire having a central core of platinum and an outer layer of nitinol.

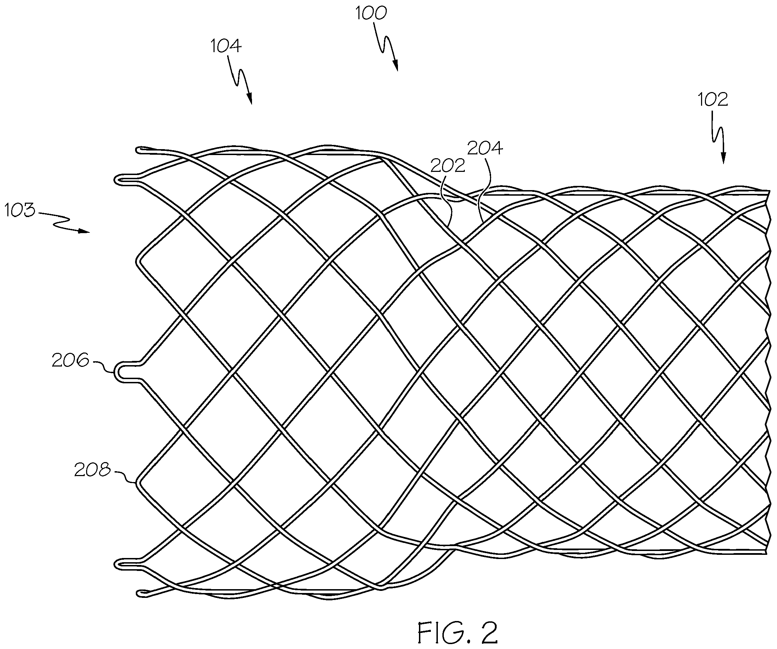

As discussed previously, in some embodiments, the stent 100 is formed by braiding one or more wires together. One such example is shown in FIG. 2, where the stent 100 is made by braiding a first wire 202 and a second wire 204.

In some embodiments, the two wires 202 and 204 are braided in a helical pattern, wherein the first wire 202 and the second wire 204 alternate with one another. The first and second wires 202 and 204 can be braided to form a variety of patterns for example, corrugated, serpentine, coil, etc.

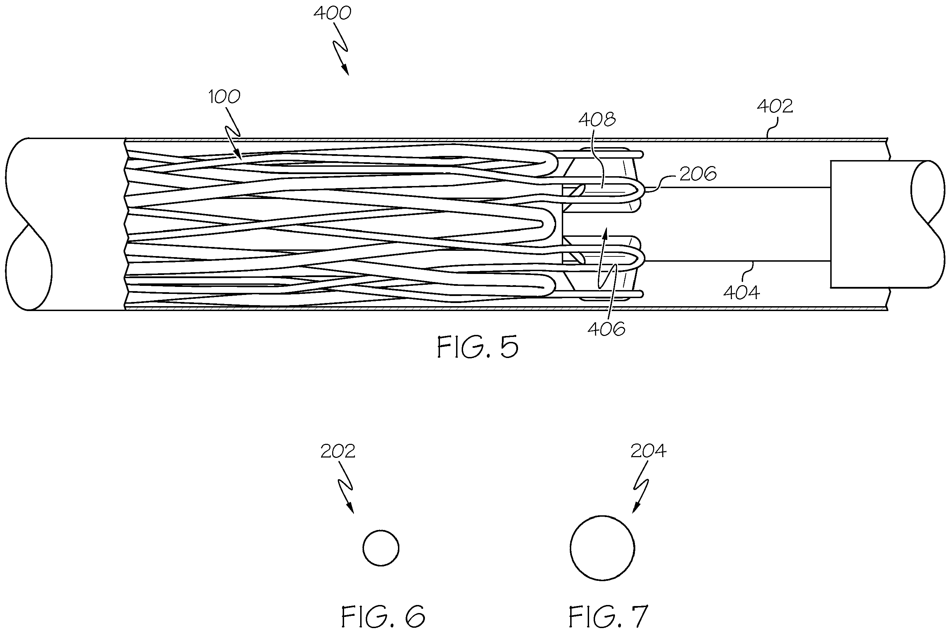

In some embodiments, for example as shown via FIGs. 6 and 7, the first wire 202 has a first cross-sectional area that is different from a second cross-sectional area of the second wire 204. In some embodiments, the first cross-sectional area is greater than the second cross-sectional area; in some other embodiments, however, the second cross-sectional area is greater than the first cross-sectional area, as depicted in FIGs. 6 and 7. Differing cross-sectional areas of the two wires 202 and 204 contributes to increased kink resistance and increased radial force of the stent 100 while yielding a stent 100 that can be sufficiently radially compressed for loading into the delivery catheter. When compared to a stent formed from wires of the same diameter, at least some embodiments of the stent 100 herein disclosed have a greater expanded radial force and are also able to be readily compressed for loading into a suitably sized and constructed delivery device. This is useful, for example, where the delivery catheter is required to be inserted through the working channel of an endoscope.

An increase in the radial force of the stent 100, when compared to stents having wires of the same diameter, is realized through comparison of the area moment of inertia, I, and the cross-sectional area, A, of such stent wires, which have a direct relation to the bending stiffness of the braided wires (e.g., 202, 204). In particular, the bending stiffness is defined as follows:

$$(1) \qquad Bending\ Stiffness = E * I$$

where E is the elastic modulus and I is the area moment of inertia.

In the instance where the braided wires (e.g., 202, 204) have a circular cross section and the wire is bent about the centroid of the wire, I is defined as:

$$(2) \qquad I = \frac{\pi}{4}r^4$$

where r is the radius of the wire.

Further, the cross-sectional area, A, of a single wire is defined as:

$$(3) \qquad A = \pi r^2$$

In light of these equations, a comparison of the following three stents is as follows, and as summarized below in Table 1.

### Stent A (PRIOR ART):

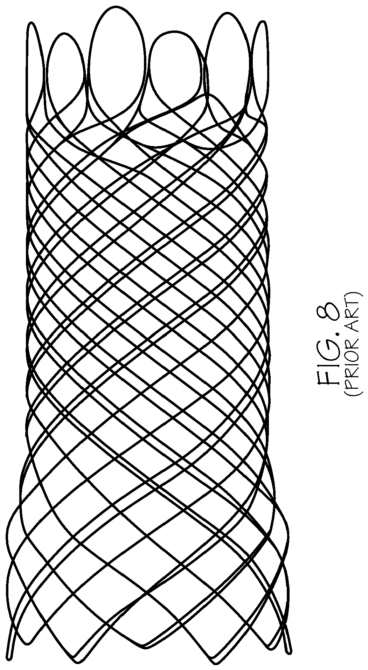

Stent A: a **prior art** stent, for example as shown in FIG. 8, formed from a 0.008" (0.203 mm) diameter wire. The cross-sectional area of two 0.008" wires is:

$$2 * A = 2\pi r^2 = 2\pi(0.004)^2 = 1.005 \mathrm{x} 10^{-4} \, \mathrm{in.}^2$$

Further, the area moment of inertia of two 0.008" wires is:

$$2 * I = 2\left(\frac{\pi}{4}\right)r^4 = 2\left(\frac{\pi}{4}\right)(0.004)^4 = 4.02124 \mathrm{x} 10^{-10} \, \mathrm{in.}^2$$

### Stent B (Example 1):

Stent B: stent formed from alternating 0.007" (0.178 mm) and 0.010" (0.254 mm) diameter wires. The cross-sectional area of one 0.007" wire and one 0.010" wire is:

$$A_1 + A_2 = \pi r_1^2 + \pi r_2^2 = \pi(0.007)^2 + \pi(0.010)^2 = 1.170 \mathrm{x} 10^{-4} \, \mathrm{in.}^2$$

Further, the area moment of inertia of one 0.007" wire and one 0.010" wire is:

$$I_1 + I_2 = \left(\frac{\pi}{4}\right)r_1^4 + \left(\frac{\pi}{4}\right)r_2^4 = \left(\frac{\pi}{4}\right)(0.007)^4 + \left(\frac{\pi}{4}\right)(0.010)^4 = 6.08733\text{x}10^{-10}\,\text{in.}^2$$

**Stent C (Example 2):**

Stent C: stent formed from alternating 0.006" (0.152 mm) and 0.011" (0.279 mm) diameter wires. The cross-sectional area of one 0.006" wire and one 0.011" wire is:

$$A_1 + A_2 = \pi r_1^2 + \pi r_2^2 = \pi(0.006)^2 + \pi(0.011)^2 = 1.233\text{x}10^{-4}\,\text{in.}^2$$

Further, the area moment of inertia of one 0.006" wire and one 0.011" wire is:

$$I_1 + I_2 = \left(\frac{\pi}{4}\right)r_1^4 + \left(\frac{\pi}{4}\right)r_2^4 = \left(\frac{\pi}{4}\right)(0.006)^4 + \left(\frac{\pi}{4}\right)(0.011)^4 = 7.82306\text{x}10^{-10}\,\text{in.}^2$$

**Table 1**

|  | $A$ (in.$^2$) (mm$^2$) | $I$(in.$^4$) (mm$^4$) |
|---|---|---|
| Stent A (Prior Art) | 1.005x10$^{-4}$ 0.0648 | 4.02124x10$^{-10}$ 1.6738x10$^{-4}$ |
| Stent B Example 1 | 1.170x10$^{-4}$ 0.0755 | 6.08733x10$^{-10}$ 2.5337x10$^{-4}$ |
| Stent C Example 2 | 1.233x10$^{-4}$ 0.0795 | 7.82306x10$^{-10}$ 3.2562x10$^{-4}$ |

As illustrated, the area moment of inertia increases to the 4th power of the wire radius while the cross-sectional area of the wire increases with the square of the wires' (202 and 204) radius. As such, although the volume occupied within the delivery device by stent B or stent C is greater than for stent A, for example, the bending stiffness of the wires increases dramatically for stents B and C in comparison to stent A. For a given length stent, the volume occupied by the stent is a function of the cross-sectional areas of the wires. The two wires 202 and 204 combine for net gains of stent radial force (compared to known stent A, for example) while still maintaining similar to only slightly higher wire cross-sectional areas. Also, because the elastic modulus, E, is a material property, it is not considered as part of the calculation because it is merely a scaling factor for a given material.

In some embodiments, the first wire 202 has a diameter of 0.007" and the second wire 204 has a diameter of 0.010". The wire sizes can be in any suitable combination, for example, 0.006" and 0.011" paired together or 0.005" (0.127 mm) and 0.012" (0.305 mm) paired together. Use of such wires in an alternating pattern provides a stent with a higher radial force while maintaining similar or the same overall cross-sectional area of (combined) wire, when compared to the PRIOR ART stent (e.g., FIG. 8, Stent A, above).

In further comparing stents A, B, and C, stent B has a 16.4% increase in wire cross-sectional area but a 51.4% increase in bending stiffness over stent A. Stent C has a 22.7% increase in wire cross-sectional area but a 95.4% increase in bending stiffness when compared to stent A.

It will further be appreciated that the stent 100 can comprise any suitable number of different sizes of diameters. For example, in some embodiments, the stent 100 has wires of three different diameters. In some embodiments, the stent 100 has wires of four, five, six, or more different diameters. Further, in some embodiments, every third, fourth, fifth, etc., wire is differently sized than the remaining wires used to form the stent.

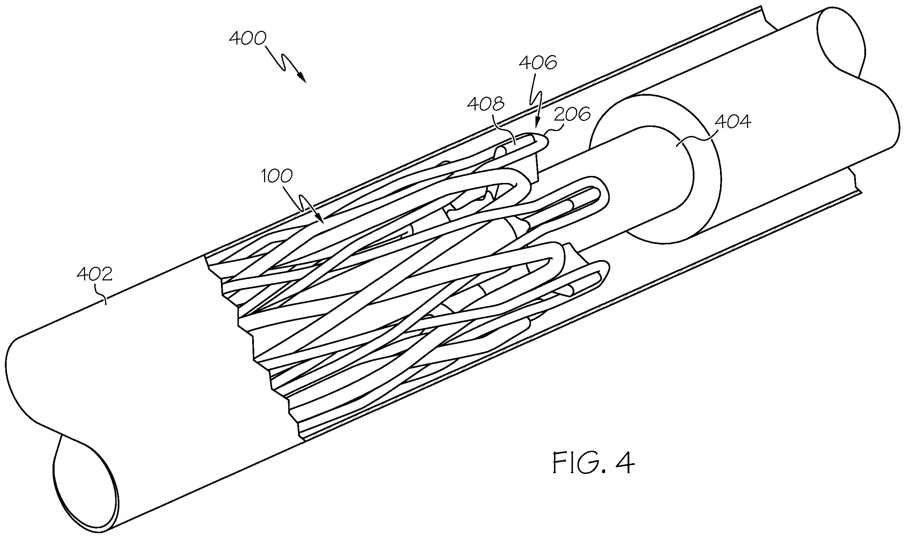

FIG. 2 is a side-view of the proximal end 104 of the stent 100 of FIG. 1. In some embodiments, the proximal end 104 of the stent 100 includes a plurality of peaks 103. The peaks 103 comprise one or more first loop ends 206 and one or more first peaks 208. The proximal end 104 of the stent 100 may include any suitable number of loop ends and peaks such as two, four, six, eight, ten, or the like. In some embodiments, the loop ends 206 are employed to removably secure the stent 100 with a delivery system for delivery of the stent 100 into the patient's body. Details of the delivery system are discussed in conjunction with FIGs. 4 and 5, below.

The first loop ends 206 and the first peaks 208 may be arranged in a circumferentially alternating pattern. In some embodiments, however, the first loop ends 206 and the first peaks 208 are arranged in another suitable pattern about the circumference of the proximal end 104 of the stent. One such example may include two loop ends 206 repeated after each first peak 208. For example, the proximal end 104 of the stent 100 may have two loop ends repeated after every single peak.

Further, although shown with respect to the proximal end 104 of the stent 100, the pattern of first loop ends 206 and first peaks 208 can also be disposed on the distal end 106.

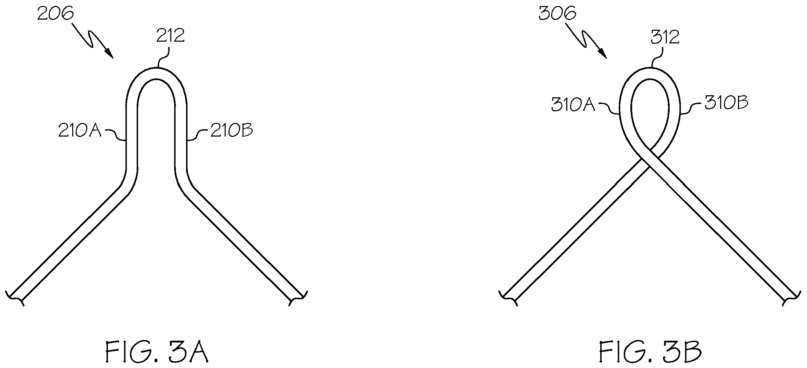

Structurally, the first loop end 206, as shown in FIG. 3A, includes a first parallel portion 210a, a second parallel portion 210b, and a bent portion 212. As shown, the two parallel portions 210a and 210b are connected to one another via the bent portion 212. At least a portion of the two parallel portions extends beyond the first peaks 208 (as shown in FIG. 2). In some embodiments, the parallel portions 210a, 210b extend parallel to one another along a longitudinal axis of the stent 100. In some embodiments, the first loop end 206 forms a crescent-shaped hook type structure suitable for engaging a portion of the delivery device.

FIG. 3B shows another example of a first loop end 306 to be used in conjunction with the stent 100 of FIGS. 1 and 2. In some embodiments, the first loop end 306 has a first curved portion 310a, a second curved portion 310b, and a bent portion 312. In contrast to the first loop end 206, the first curved portion 310a intersects with the second curved portion 310b while being connected to one another via the bent portion 312. The first loop end 306 thus forms an oval-shaped closed hook structure suitable for engaging a portion of the delivery device.

Turning to FIGs. 4 and 5, a delivery catheter 400 includes a retractable sheath 402, an inner catheter member 404, and an anchor member 406. In some embodiments, the inner catheter member 404 is slidably disposed within a lumen (not shown) of the retractable sheath 402. Further, the inner catheter member 404 is employed to receive and deploy the stent 100 into a body lumen of a patient.

To accomplish this, the anchor member 406 is disposed exteriorly to the inner catheter member 404, where the anchor member 406 includes a plurality of protrusions 408 extending radially therefrom. A proximal end of the stent 100 is coupled to the anchor member 406 such that the first loop ends (206 and 306 of FIGS. 3A and 3B) are engaged with the protrusions 408 of the anchor member 406. Engagement of the stent 100 and the anchor member 406 allows a physician to advance the delivery catheter 400 inside the body lumen. Once a target location within the body lumen is reached, the physician manipulates the delivery catheter 400 by retracting the retractable sheath 402. Upon retraction of the retractable sheath 402, the distal end 106 of the stent 100 starts to expand, for example where the stent is self-expanding. During partial expansion, however, before the retractable sheath 402 is approximately 90% completely retracted, for example, the proximal end 104 of the stent 100 remains engaged to the protrusions 408 so that the retractable sheath 408 can be moved distally relative to the stent 100, allowing the distal end 106 of the stent 100 to be reconstrained within the retractable sheath 402 and repositioned. Upon approximately 90% retraction of the retractable sheath 402, however, the proximal end 104 of the stent begins to disengage from the protrusions 408 and the stent 100 can no longer be retracted and repositioned.

Once the stent is deployed, the delivery catheter, along with the inner catheter member 404, is removed from the body lumen.

With reference to FIG. 5, due to staggering the first peaks 208 with the first loop ends 206, the stent 100 takes on a reduced unexpanded profile when compared to a stent having peaks 103 (FIG. 2) that terminate at the same longitude as one another. In particular, due to the presence of the anchor member 406 and the tight bends of the wires at the peaks 103, it has been found that offsetting the peaks 103 (e.g., via staggering first peaks 208 and first loop ends 206) reduces the profile of the stent 100 in the unexpanded configuration. This way, not all of the peaks 103 overlap with the protrusions 408; instead only the first loop ends 206 overlap with the protrusions 408 while the first peaks 208 are longitudinally offset. Moreover, a reduction of the profile of stent 100 in its unexpanded configuration allows for use of a delivery catheter 400 than with known configurations.

When used in combination with the alternating sized wires (e.g., first and second wires 202, 204), the stent 100 having staggered first peaks 208 and first loop ends 206 provides greater radial strength yet can be introduced via a similarly sized, or even smaller, catheter 400 than known stent/catheter combinations.

In some embodiments, the first wire 202 is used to form the first loop ends 206 and the second wire 204 is used to form the first peaks such that the smaller diameter wire (e.g., first wire 202) is placed over the protrusions 408. Such a configuration provides a stent 100 and catheter 400 combination having a further reduced delivery profile.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

**Claims**

1.  A stent comprising:

    a braided wire body having a proximal end and a distal end, the braided wire body formed from at least a first wire and at least a second wire, the first wire having a first cross-sectional area and the second wire having a second cross-sectional area different from the first cross-sectional area; wherein the proximal end of the braided wire body comprises a plurality of peaks, the peaks comprising first peaks and first loop ends, the first loop ends circumferentially alternating with the first peaks, the first loop ends extending proximally beyond the first peaks, **characterised in that** the first loop ends each include a first parallel portion and a second parallel portion and a bent portion, the two parallel portions being connected to one another via the bent portion, and with at least a portion of the first and second parallel portions extending beyond the first peaks.

2.  The stent of claim 1, wherein the cross-sectional area of the second wire is greater than the cross-sectional area of the first wire.

3.  The stent of any one of the preceding claims, wherein the first and second wires are arranged in a helical pattern.

4.  The stent of any one of the preceding claims, wherein the first and second wires alternate with one another.

5.  The stent of any one of the preceding claims, wherein the second wire has a cross sectional diameter between 0.178 mm (0.007") and 0.305 mm (0.012").

6.  The stent of any one of the preceding claims, wherein the first wire has a cross sectional diameter between 0.127 mm (0.005") and 0.178 mm (0.007").

7.  The stent of any one of the preceding claims, wherein the second wire has a cross-sectional area of between 0.0248 square mm (0.000038 square inches) and 0.0774 square mm (0.00012 square inches).

8.  The stent of any one of the preceding claims, wherein the first wire has a cross-sectional area of between 0.0122 square mm (0.000019 square inches) and 0.0251 square mm (0.00039 square inches).

9.  The stent of any one of the preceding claims, wherein at least one of the first and second wires is a composite wire.

10. The stent of claim 9, wherein the composite wire includes nitinol.

11. The stent of claim 9, wherein the composite wire includes a radiopaque core.

12. A catheter-and-stent delivery system comprising:

    a stent having a proximal end and a distal end, the proximal end of the stent comprising a plurality of peaks, the peaks comprising first peaks and first loop ends, the first loop ends circumferentially alternating with the first peaks, the first loop ends extending proximally beyond the first peaks;
    a delivery catheter comprising a retractable sheath, an inner catheter member, and an anchor member, the anchor member disposed exteriorly to the inner catheter member and comprising a plurality of protrusions, the protrusions extending into the first loop ends of the stent; the retractable sheath surrounding the stent and anchor member,
    **characterised in that** said stent's first loop ends each include a first parallel portion and a second parallel portion and a bent portion, the two parallel portions being connected to one another via the bent portion and with at least a portion of the first and second parallel portions extending beyond the first peaks.

13. The delivery catheter and stent of claim 12, the stent further comprising first and second wires, the first wire having a first cross-sectional area and the second wire having a second cross-sectional area different from the first cross-sectional area.

14. The delivery catheter and stent of claim 13, wherein the first wire defines the first loop ends and the second wire defines the first peaks, the second cross-sectional area being greater than the first cross-sectional area.

**15.** The delivery catheter and stent of claim 12, 13, or 14, wherein the stent defines a longitudinal axis and the parallel portions of the stent's first loop ends extend parallel to the longitudinal axis.

**Patentansprüche**

**1.** Stent, der aufweist:

einen Flechtdrahtkörper mit einem proximalen Ende und einem distalen Ende, wobei der Flechtdrahtkörper aus mindestens einem ersten Draht und mindestens einem zweiten Draht gebildet ist, der erste Draht eine erste Querschnittsfläche hat und der zweite Draht eine zweite Querschnittsfläche hat, die sich von der ersten Querschnittsfläche unterscheidet; wobei das proximale Ende des Flechtdrahtkörpers mehrere Spitzen aufweist, wobei die Spitzen erste Spitzen und erste Schlaufenenden aufweisen, wobei sich die ersten Schlaufenenden mit den ersten Spitzen entlang des Umfangs abwechseln, wobei sich die ersten Schlaufenenden über die ersten Spitzen hinaus proximal erstrecken,
**dadurch gekennzeichnet, dass** die ersten Schlaufenenden jeweils einen ersten Parallelabschnitt und einen zweiten Parallelabschnitt sowie einen gebogenen Abschnitt aufweisen, wobei die beiden Parallelabschnitte über den gebogenen Abschnitt miteinander verbunden sind und sich mindestens ein Abschnitt des ersten und zweiten Parallelabschnitts über die ersten Spitzen hinaus erstreckt.

**2.** Stent nach Anspruch 1, wobei die Querschnittsfläche des zweiten Drahts größer als die Querschnittsfläche des ersten Drahts ist.

**3.** Stent nach einem der vorstehenden Ansprüche, wobei der erste und zweite Draht in einem Spiralmuster angeordnet sind.

**4.** Stent nach einem der vorstehenden Ansprüche, wobei sich der erste und zweite Draht miteinander abwechseln.

**5.** Stent nach einem der vorstehenden Ansprüche, wobei der zweite Draht einen Querschnittsdurchmesser zwischen 0,178 mm (0,007") und 0,305 mm (0,012") hat.

**6.** Stent nach einem der vorstehenden Ansprüche, wobei der erste Draht einen Querschnittsdurchmesser zwischen 0,127 mm (0,005") und 0,178 mm (0,007") hat.

**7.** Stent nach einem der vorstehenden Ansprüche, wobei der zweite Draht eine Querschnittsfläche zwischen 0,0248 mm$^2$ (0,000038 Inch$^2$) und 0,0774 mm$^2$ (0,00012 Inch$^2$) hat.

**8.** Stent nach einem der vorstehenden Ansprüche, wobei der erste Draht eine Querschnittsfläche zwischen 0,0122 mm$^2$ (0,000019 Inch$^2$) und 0,0251 mm$^2$ (0,00039 Inch$^2$) hat.

**9.** Stent nach einem der vorstehenden Ansprüche, wobei der erste und/oder zweite Draht ein Verbunddraht ist.

**10.** Stent nach Anspruch 9, wobei der Verbunddraht Nitinol aufweist.

**11.** Stent nach Anspruch 9, wobei der Verbunddraht einen röntgendichten Kern aufweist.

**12.** Katheter-Stent-Abgabesystem, das aufweist:

einen Stent mit einem proximalen Ende und einem distalen Ende, wobei das proximale Ende des Stents mehrere Spitzen aufweist, die Spitzen erste Spitzen und erste Schlaufenenden aufweisen, wobei sich die ersten Schlaufenenden mit den ersten Spitzen entlang des Umfangs abwechseln, wobei sich die ersten Schlaufenenden über die ersten Spitzen hinaus proximal erstrecken;
einen Abgabekatheter mit einer zurückziehbaren Hülle, einem Innenkatheterteil und einem Ankerteil, wobei das Ankerteil außerhalb des Innenkatheterteils angeordnet ist und mehrere Vorsprünge aufweist, wobei sich die Vorsprünge in die ersten Schlaufenenden des Stents erstrecken; die zurückziehbare Hülle den Stent und das Ankerteil umgibt,
**dadurch gekennzeichnet, dass** die ersten Schlaufenenden des Stents jeweils einen ersten Parallelabschnitt und einen zweiten Parallelabschnitt sowie einen gebogenen Abschnitt aufweisen, wobei die beiden Parallelabschnitte über den gebogenen Abschnitt miteinander verbunden sind und sich mindestens ein Abschnitt des ersten und zweiten Parallelabschnitts über die ersten Spitzen hinaus erstreckt.

**13.** Abgabekatheter und Stent nach Anspruch 12, wobei der Stent ferner einen ersten und einen zweiten Draht aufweist, der erste Draht eine erste Querschnittsfläche hat und der zweite Draht eine zweite Querschnittsfläche hat, die sich von der ersten Querschnittsfläche unterscheidet.

**14.** Abgabekatheter und Stent nach Anspruch 13, wobei der erste Draht die ersten Schlaufenenden bildet und der zweite Draht die ersten Spitzen bildet, wobei die zweite Querschnittsfläche größer als die erste Querschnittsfläche ist.

**15.** Abgabekatheter und Stent nach Anspruch 12, 13 oder 14, wobei der Stent eine Längsachse bildet und sich die Parallelabschnitte der ersten Schlaufenenden des Stents parallel zur Längsachse erstrecken.

**Revendications**

**1.** Stent comprenant:

un corps de fils tressés ayant une extrémité proximale et une extrémité distale, le corps de fils tressés formé d'au moins un premier fil et d'au moins un second fil, le premier fil ayant une première aire en section transversale et le second fil ayant une seconde aire en section transversale différente de la première aire en section transversale; dans lequel l'extrémité proximale du corps de fils tressés comprend une pluralité de pics, les pics comprenant des premiers pics et des premières extrémités de boucle, les premières extrémités de boucle alternant circonférentiellement avec les premiers pics, les premières extrémités de boucle s'étendant de manière proximale au-delà des premiers pics,
**caractérisé en ce que** les premières extrémités de boucle incluent chacune une première partie parallèle et une seconde partie parallèle et une partie pliée, les deux parties parallèles étant reliées l'une à l'autre via la partie pliée, et avec au moins une partie des première et seconde parties parallèles s'étendant au-delà des premiers pics.

**2.** Stent selon la revendication 1, dans lequel l'aire en section transversale du second fil est supérieure à l'aire en section transversale du premier fil.

**3.** Stent selon l'une quelconque des revendications précédentes, dans lequel les premier et second fils sont agencés selon un motif hélicoïdal.

**4.** Stent selon l'une quelconque des revendications précédentes, dans lequel les premier et second fils alternent l'un avec l'autre.

**5.** Stent selon l'une quelconque des revendications précédentes, dans lequel le second fil a un diamètre en section transversale entre 0,178 mm (0,007") et 0,305 mm (0,012").

**6.** Stent selon l'une quelconque des revendications précédentes, dans lequel le premier fil a un diamètre en section transversale entre 0,127 mm (0,005") et 0,178 mm (0,007").

**7.** Stent selon l'une quelconque des revendications précédentes, dans lequel le second fil a une aire en section transversale entre 0,0248 mm carré (0,000038 pouce carré) et 0,0774 mm carré (0,00012 pouce carré).

**8.** Stent selon l'une quelconque des revendications précédentes, dans lequel le premier fil a une aire en section transversale entre 0,0122 mm carré (0,000019 pouce carré) et 0,0251 mm carré (0,00039 pouce carré).

**9.** Stent selon l'une quelconque des revendications précédentes, dans lequel au moins l'un des premier et second fils est un fil composite.

**10.** Stent selon la revendication 9, dans lequel le fil composite inclut du nitinol.

**11.** Stent selon la revendication 9, dans lequel le fil composite inclut une âme radio-opaque.

**12.** Système de mise en place de cathéter et de stent comprenant:

un stent ayant une extrémité proximale et une extrémité distale, l'extrémité proximale du stent comprenant une pluralité de pics, les pics comprenant des premiers pics et des premières extrémités de boucle, les premières extrémités de boucle alternant circonférentiellement avec les premiers pics, les premières extrémités de boucle s'étendant de manière proximale au-delà des premiers pics;
un cathéter de mise en place comprenant une gaine rétractable, un élément de cathéter interne et un élément d'ancrage, l'élément d'ancrage disposé extérieurement par rapport à l'élément de cathéter interne et comprenant une pluralité de saillies, les saillies s'étendant dans les premières extrémités de boucle du stent; la gaine rétractable entourant le stent et l'élément d'ancrage, **caractérisé en ce que** lesdites premières extrémités de boucle du stent incluent chacune une première partie parallèle et une seconde partie parallèle et une partie pliée, les deux parties parallèles étant reliées l'une à l'autre via la partie pliée et avec au moins une partie des première et seconde parties parallèles s'étendant au-delà des premiers pics.

**13.** Cathéter de mise en place et stent selon la revendication 12, le stent comprenant en outre des premier et second fils, le premier fil ayant une première aire en section transversale et le second fil ayant une seconde aire en section transversale différente de la première aire en section transversale.

**14.** Cathéter de mise en place et stent selon la revendication 13, dans lesquels le premier fil définit les premières extrémités de boucle et le second fil définit les premiers pics, la seconde aire en section transversale étant supérieure à la première aire en section transversale.

**15.** Cathéter de mise en place et stent selon la revendication 12, 13 ou 14, dans lesquels le stent définit un axe longitudinal et les parties parallèles des premières extrémités de boucle du stent s'étendent parallèlement à l'axe longitudinal.

100

104 102 106

FIG. 1

FIG. 2

EP 3 128 959 B1

312

310B

310A

306

FIG. 3B

212

210B

210A

206

FIG. 3A

FIG. 4

FIG. 5

FIG. 6

FIG. 7

EP 3 128 959 B1

FIG. 8
(PRIOR ART)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006053270 A2
- DE 102011011180 A1

- US 20060116752 A